# FASCICULE DE BREVET EUROPEEN

(11) **EP 4 182 684 B1**
(45) Date de publication et mention de la délivrance du brevet: **24.04.2024**
(21) Numéro de dépôt: 21743113.9
(22) Date de dépôt: 07.07.2021
(51) Int. Cl.: G01N 33/00, A01G 7/00

(54) **SYSTEME D'ANALYSE ELECTRO-PHYSIOLOGIQUE DE PLANTES**
SYSTEM ZUR ELEKTROPHYSIOLOGISCHEN ANALYSE VON PFLANZEN
SYSTEM FOR ELECTROPHYSIOLOGICAL ANALYSIS OF PLANTS

(30) Priorité: 15.07.2020 FR 2007385; 15.07.2020 FR 2007386; 15.07.2020 FR 2007387
(43) Date de publication de la demande: 24.05.2023
(73) Titulaire: Vegetal Signals, 33000 Bordeaux (FR)
(72) Inventeur: COCHETEUX, Patrice, 33300 BORDEAUX (FR); LE BOURDIEC, Fabien, 33300 BORDEAUX (FR)
(74) Mandataire: Aquinov
(86) Numéro de dépôt international: PCT/EP2021/068889
(87) Numéro de publication internationale: WO 2022/013040

(56) Documents cités:
- WO-A1-2017/032750
- BE-A- 830 574
- DE-B3-102006 041 127
- US-A- 3 967 198
- COMPARINI DIEGO ET AL: "Stem electrical properties associated with water stress conditions in olive tree", AGRICULTURAL WATER MANAGEMENT, ELSEVIER, AMSTERDAM, NL, vol. 234, 17 mars 2020 (2020-03-17), XP086106748, ISSN: 0378-3774, DOI: 10.1016/J.AGWAT.2020.106109 [extrait le 2020-03-17]

## Description

L'invention concerne le domaine de l'analyse électro-physiologique des plantes. Plus précisément, l'invention concerne un système d'analyse électro-physiologique de plusieurs plantes.

Il a été observé que les plantes émettent des signaux électriques, notamment sous la forme de potentiels d'action, traduisant son état physiologique. En mesurant ces signaux électriques au moyen d'électrodes et en les analysant, il est ainsi possible de déterminer l'état physiologique d'une plante de façon plus rapide et plus fiable que par des moyens d'observation directe. En outre, en cas de détection d'une situation de stress ou de maladie, il est également possible de proposer, de façon immédiate et automatique, des actions correctives permettant d'y remédier.

Toutefois, les systèmes d'analyse électro-physiologique des plantes existants sont conçus pour une utilisation en laboratoire et ne conviennent pas pour un usage agricole. En effet, les systèmes connus reposent, d'une part, sur un système d'acquisition de signaux électriques permettant de numériser ces signaux en vue de leur traitement et d'autre part sur un module de traitement de ces signaux numérisés implémentant notamment des méthodes d'analyses statistiques. Il est donc nécessaire d'acquérir un grand nombre de signaux électriques à partir de plusieurs plantes, de façon simultanée et sur de longues durées. Ce passage à l'échelle d'un champ implique une pluralité de contraintes. En premier lieu, il est nécessaire de déployer une pluralité de points de mesure, notamment plusieurs centaines de points de mesure, sur une grande surface, notamment sur plusieurs milliers de mètres carrés. Il est ainsi nécessaire que l'ensemble de ces points de mesures puisse être déployé facilement et soit conçu de façon simple pour présenter un coût d'installation et d'exploitation réduit.

Dans un deuxième temps, il est nécessaire de pouvoir alimenter l'électronique permettant d'acquérir et de numériser les signaux électriques obtenus au niveau de ces points de mesures. Or, cette multiplicité de points de mesures contraint dès lors à employer une multitude de câbles d'alimentation pour alimenter électriquement chacun des dispositifs d'acquisition et de câbles de transmission de données pour permettre la transmission des signaux numérisés vers le module de traitement. Le nombre de câbles ainsi nécessaires nuit à la robustesse du système, à son coût et à sa simplicité d'installation et d'utilisation.

Enfin, les signaux électriques émis par les plantes présentent une très faible intensité, de l'ordre du millivolt. Or, l'alimentation électrique fournit à l'électronique d'acquisition et de numérisation est susceptible de perturber, voire d'influencer de façon néfaste cette acquisition et cette numérisation, ce qui pourrait fausser la mesure et les données.

DE102006041127décrit un exemple d'un système connu pour l'analyse électro-physiologique des plantes.

La présente invention se place ainsi dans ce contexte et a ainsi pour but de répondre aux besoins cités tout en remédiant aux inconvénients cités.

A ces fins, l'invention a pour objet un système d'analyse électro-physiologique de plantes, caractérisé en ce qu'il comporte :
a. Une pluralité de systèmes d'acquisition, chaque système d'acquisition comportant :
   i. une pluralité de paires d'électrodes comportant chacune une première électrode et d'une deuxième électrode destinées à être connectée chacune à une même plante pour capter des premier et deuxième potentiels électriques analogiques;
   ii. une pluralité d'amplificateurs opérationnels agencés pour mesurer les différences de potentiel entre les premiers et deuxièmes potentiels électriques analogique acquis par les premières et deuxièmes électrodes de ce système d'acquisition et une pluralité de convertisseurs analogique numérique agencés pour convertir lesdites différences de potentiel en des signaux numériques ;
b. Un système central de collecte de données comportant une mémoire et une source d'énergie électrique ;
   et en ce que chaque système d'acquisition est relié au système central et comporte :
   c. une unité de raccordement agencée pour transmettre lesdits signaux numériques au système central ;
   d. une unité de puissance agencée pour transmettre une puissance électrique obtenue de ladite source d'énergie électrique aux amplificateurs opérationnels et aux convertisseurs analogiques numériques ;
      et dans lequel chaque système d'acquisition est agencé pour isoler galvaniquement les amplificateurs opérationnels et les convertisseurs analogique numérique de l'unité de puissance et de l'unité de raccordement.

Selon l'invention, un système central est agencé d'une part pour collecter les données issues des systèmes d'acquisition et pour transmettre une alimentation électrique à chacun de ces systèmes d'acquisition. Cette architecture en étoile, organisée autour de ce système central, permet d'augmenter sensiblement le nombre de points de mesure tout en réduisant le nombre de câbles et de centraliser la source d'énergie électrique. Les points de mesure et l'électronique active de chaque système d'acquisition peuvent alors être conçus et organisés selon la topologie convenant le mieux au champ dans lequel ils doivent être déployés, chaque système d'acquisition garantissant que l'alimentation électrique fournie par le système central soit transmise à cette électronique active tout en évitant de parasiter ou d'influencer les données générées par cette électronique active.

De préférence, chaque paire d'électrodes peut être connectée à une plante de sorte à mesurer un champ électrique linéaire généré par la superposition des dépolarisations transitoires des membranes des cellules foliaires et/ou par les échanges ioniques au niveau du système racinaire. Par exemple, chacune des première et deuxième électrodes peut être connectée à la tige de la plante pour être en contact avec le phloème ou en variante, l'une des électrodes peut être connectée à la tige de la plante et l'autre des électrodes peut être connectée à un bulbe souterrain de la plante. Si on le souhaite, chaque électrode est une électrode de type aiguille. Ce type d'électrode est particulièrement adapté à un usage agricole, la connexion d'une électrode de type aiguille à la plante étant plus robuste que celle avec une électrode de type cupule dans un contexte où les plantes sont susceptibles de croitre et de subir des contraintes mécaniques, notamment du fait du vent.

Si on le souhaite, chaque deuxième électrode d'un système d'acquisition est reliée à un potentiel électrique commun de référence, notamment une masse d'une partie du système d'acquisition, isolé de l'unité de puissance et de l'unité de raccordement.

Avantageusement, chaque système d'acquisition comporte une pluralité de terminaux reliés chacun à un groupe de paires d'électrodes de ce système d'acquisition et agencés pour collecter lesdites potentiels électriques analogiques captées par les premières et deuxièmes électrodes de ce groupe, et un relai relié auxdits terminaux de ce système d'acquisition et au système central, ce relai comportant ladite unité de raccordement et ladite unité de puissance. Le cas échéant, les amplificateurs opérationnels et les convertisseurs analogique numérique de chaque système d'acquisition sont prévus dans le relai et/ou dans les terminaux de ce système d'acquisition, et le relai de chaque système d'acquisition est agencé pour isoler galvaniquement ces amplificateurs opérationnels et ces convertisseurs analogique numérique de ce système d'acquisition de son unité de puissance et de son unité de raccordement. Dans ce mode de réalisation, chaque système d'acquisition présente ainsi une topologie en arbre, dont la racine est formée par le relai et dont les branches s'étendent jusqu'au paires d'électrodes. Pour chaque système d'acquisition, on pourra prévoir indifféremment que les terminaux soient reliés directement au relai, l'arbre présentant dans ce cas seulement deux niveaux, ou au contraire que les terminaux soient reliés par des extenseurs, afin d'atteindre des points de mesure plus éloignés, ou par des concentrateurs ou des hubs, afin de démultiplier le nombre de terminaux, l'arbre pouvant dans ce cas présenter plus de deux niveaux.

De préférence, chaque deuxième électrode de chaque système d'acquisition est reliée à un potentiel électrique commun de référence, notamment une masse du relai de ce système d'acquisition, isolée de l'unité de puissance et de l'unité de raccordement de ce relai.

Si on le souhaite, chaque première électrode est associée à un filtre agencé pour filtrer le premier potentiel électrique analogique capté par cette première électrode. Le ou chacun des filtres pourra comporter un filtre de lissage analogique et/ou un filtre de protection contre les décharges électrostatiques.

Dans un mode de réalisation de l'invention, chaque terminal de chaque système d'acquisition est agencé pour transmettre les potentiels électriques analogiques qu'il collecte vers le relai de ce système d'acquisition. Le cas échéant, le relai de chaque système d'acquisition comporte une pluralité d'étages d'acquisition chacun relié au moins à l'un des terminaux de ce système d'acquisition pour recevoir lesdits potentiels électriques analogiques collectés et comportant au moins un amplificateur opérationnel et un convertisseur analogique numérique pour mesurer lesdites différences de potentiel et convertir lesdites différences de potentiel en lesdits signaux numériques, chaque étage d'acquisition étant relié à l'unité de raccordement et l'unité de puissance en étant isolé galvaniquement de cette unité de puissance et de son unité de raccordement. Dans ce mode de réalisation, les terminaux peuvent être formés par des borniers auxquels sont reliés les paires d'électrodes, l'électronique active de chaque système d'acquisition étant embarquée dans le relai de ce système d'acquisition. On optimise ainsi le coût des points de mesure. On pourra prévoir que chaque étage d'acquisition d'un relai soit relié à un ou plusieurs terminaux.

De préférence, chaque deuxième électrode de chaque système d'acquisition est reliée à un potentiel électrique commun de référence, notamment une masse, d'un étage d'acquisition du relai de ce système d'acquisition auquel est reliée cette deuxième électrode.

On pourra prévoir que chaque étage d'acquisition comporte un convertisseur analogique numérique multivoies comportant une pluralité d'amplificateurs opérationnels et une pluralité de convertisseurs analogiques numériques élémentaire, chaque amplificateur opérationnel étant destiné à recevoir les potentiels électriques analogiques acquis par une paire d'électrodes d'un des groupes d'électrodes et étant agencé pour mesurer la différence de potentiel entre ces potentiels électriques analogiques et chaque convertisseur analogique numérique élémentaire étant agencé pour convertir l'une de ces différences de potentiel en un signal numérique. Parexemple, chaque convertisseur analogique numérique élémentaire pour être un convertisseur analogique numérique élémentaire delta-sigma relié à la sortie de l'un des amplificateurs opérationnels. On entend par liaison entre la sortie d'un amplificateur opérationnel et un convertisseur analogique numérique élémentaire delta-sigma aussi bien une liaison simple entre un amplificateur à sortie simple et le convertisseur sur lequel transite le signal amplifié par l'amplificateur qu'une liaison double entre un amplificateur à sortie différentielle et le convertisseur où le signal amplifié est obtenu par la différence entre les signaux transitant sur chacune des lignes de la liaison double.

Par exemple, chaque amplificateur opérationnel pourra être un amplificateur à gain programmable (également appelé PGA de l'anglais Programmable Gain Amplifier) de type faible bruit. Le cas échéant, chaque amplificateur opérationnel pourra être agencé pour présenter un gain d'au maximum 24 et pour introduire un bruit dont le niveau moyen est inférieur à 5 µV et notamment sensiblement de 0,1µV dans le cas d'un amplificateur de gain de 24. Avantageusement, chaque convertisseur analogique numérique élémentaire delta-sigma est agencé pour présenter un taux d'échantillonnage supérieur à 50 SPS (sample per second) ou de 50Hz, voir supérieur à 250 SPS ou 250 Hz, et pour convertir un signal reçu en entrée en un signal numérique codé sur 24 bits. Le cas échéant, l'ensemble unitaire formé par un amplificateur opérationnel et un convertisseur analogique numérique élémentaire delta-sigma est agencé pour présenter un nombre de bits effectifs (également appelé ENOB, de l'anglais Effective Number Of Bits) d'au moins 16, et notamment d'au moins 19 pour un gain de l'amplificateur de 24 et pour un taux d'échantillonnage de 250 SPS. Cette combinaison est particulièrement adaptée pour l'analyse électro-physiologique des plantes dans la mesure où les différences de potentiel électrique issues des amplificateurs opérationnels sont des signaux analogiques de faible amplitude (de l'ordre du microvolt) qui peuvent être amplifiés par l'amplificateur en introduisant peu de bruit et de distorsion, grâce à un faible gain, et qui pourtant être convertis par le convertisseur en des signaux numériques avec une haute résolution (de l'ordre du nanovolt).

Avantageusement, chaque étage d'acquisition du relai de chaque système d'acquisition est relié à une pluralité de terminaux de ce système d'acquisition pour recevoir lesdits potentiels électriques analogiques collectés par ces terminaux, ledit étage comportant un multiplexeur agencé pour multiplexer lesdits premiers potentiels électriques analogiques collectés par lesdits terminaux. Le cas échéant, le multiplexeur de chaque étage d'acquisition est agencé pour transmettre les premiers potentiels électriques analogiques multiplexés vers le convertisseur analogique numérique multivoies. L'utilisation d'un multiplexeur permet d'accroitre le nombre de terminaux gérés par un même relai, et donc de démultiplier le nombre de points de mesure.

Dans un autre mode de réalisation de l'invention, chaque terminal de chaque système d'acquisition comporte au moins un amplificateur opérationnel pour mesurer lesdites différences de potentiel entre les potentiels électriques analogiques qu'il collecte et pour transmettre ces différences de potentiel vers le relai de ce système d'acquisition. Le cas échéant, le relai de chaque système d'acquisition comporte une pluralité d'étages d'acquisition chacun relié au moins à l'un des terminaux de ce système d'acquisition pour recevoir lesdits différences de potentiels électriques analogiques, chaque étage d'acquisition étant relié à l'unité de raccordement et l'unité de puissance en étant isolé galvaniquement de cette unité de puissance et de son unité de raccordement. On pourra prévoir que les convertisseurs analogique numérique soient agencés dans les terminaux, ou soient embarqués dans les relais. Dans ce mode de réalisation, bien que le coût des points de mesure augmente, on s'assure que la mesure et l'amplification des différences de potentiels électriques soient réalisées au plus proche des plantes, de sorte que les pertes induites par la transmission par câble de ces différences de potentiels électriques, depuis les terminaux vers les relais, soient négligeables et n'influencent pas les données transmises ensuite au système central. De même, il est ainsi possible de rendre négligeable un bruit qui se propagerait sur l'un de ces câbles, tel qu'une fuite de courant ou un bruit répercuté depuis un autre câble.

De préférence, l'unité de raccordement comporte un microcontrôleur agencé pour sérialiser les signaux numériques convertis par les convertisseurs analogique numérique et transmis par chacun des étages d'acquisition à l'unité de raccordement en un unique signal numérique et pour transmettre cet unique signal numérique au système central. Grâce à ce microcontrôleur, on peut transmettre les données issues des convertisseurs analogique numérique de chaque relai au système central par un seul et même câble. En variante, on pourrait remplacer ce microcontrôleur par un circuit intégré agencé pour réaliser cette sé-rialisation, notamment par un FPGA.

Dans un mode de réalisation de l'invention, le système central est relié à chaque système d'acquisition par un réseau de transmission de données, le système central étant agencé pour transmettre à chaque système d'acquisition des signaux contenant une puissance électrique, notamment générée par la source d'énergie électrique, via le réseau de transmission de données. Le cas échéant, l'unité de raccordement de chaque système d'acquisition est agencée pour transmettre lesdits signaux numériques au système central via ledit réseau de transmission de données, et l'unité de puissance de chaque système d'acquisition est agencée pour extraire ladite puissance électrique depuis les signaux reçus du système central via le réseau de transmission de donnée et pour transmettre ladite puissance électrique extraite aux amplificateurs opérationnels et aux convertisseurs analogiques numériques. Le cas échéant, l'unité de raccordement et l'unité de puissance peuvent former une même unité de raccordement au réseau de transmission de données. Grâce à ces caractéristiques, il est possible de mutualiser au sein d'un même câble, reliant un système d'acquisition au système central, les fonctions d'alimentation électrique et de transmission de données. Dès lors, le nombre de câbles nécessaires à l'exploitation du système d'analyse peut être réduit, ce qui simplifie l'installation et l'utilisation de ce système tout en augmentant sa robustesse, notamment en diminuant le nombre de connecteurs nécessaires.

Avantageusement, l'unité de raccordement au réseau de chaque système d'acquisition comporte un connecteur pour être reliée au système central par un câble dudit réseau de transmission de données, par exemple de type Ethernet, implémentant une fonctionnalité de transmission de puissance électrique, par exemple de type POE (de l'anglais Power Over Ethernet), et notamment apte à délivrer une puissance électrique d'au moins 10 Watts. Un tel câble de ce type de réseau de transmission de données comporte au moins deux fils, par exemple quatre paires de fils torsadées dans le cas d'un câble Ethernet de type RJ-45, les données transitant par au moins l'un des fils, notamment par au moins deux fils, les données étant transmises par la différence de potentiels sur les deux fils, et la puissance électrique par au moins deux fils, notamment par au moins deux paires de fils, l'une des paires fournissant un pôle négatif pour ladite puissance électrique et l'autre des paires fournissant un pôle positif pour ladite puissance électrique. Dans un exemple, la paire de fils transmettant les données peut être l'une des paires de fils transmettant l'un des pôles pour ladite puissance électrique. En variante, la paire de fils transmettant les données peut être une paire de fils distincte des paires de fils transmettant les pôles pour ladite puissance électrique.

Dans un mode de réalisation de l'invention, l'unité de raccordement au réseau de chaque système d'acquisition comporte un diviseur comprenant au moins deux transformateurs agencés pour émettre et/ou recevoir des signaux du réseau de transmission de données, le diviseur étant agencé pour extraire ladite puissance électrique depuis lesdits transformateurs. Il peut par exemple s'agir de transformateurs d'isolement. Le cas échéant, l'un des transformateurs peut être destinée à une liaison montante pour transmettre des données issues du système central vers l'unité de raccordement au réseau, l'enroulement primaire de ce transformateur étant destiné à être connecté à l'un des fils, par exemple à une paire torsadée de fils, du câble du réseau de transmission de données. L'autre des transformateurs peut être destinée à une liaison descendante pour transmettre le ou les signaux numériques générés par le système d'acquisition vers le système central, l'enroulement secondaire de ce transformateur étant destiné à être connecté à un autre des fils, par exemple à une autre paire torsadée de fils, du câble du réseau de transmission de données. Avantageusement, l'unité de raccordement au réseau est agencée pour extraire la puissance électrique depuis l'enroulement primaire du transformateur montant et depuis l'enroulement secondaire du transformateur descendant.

Avantageusement, l'unité de raccordement au réseau de chaque système d'acquisition comporte une interface de conversion analogique/numérique et de modulation/démodulation du ou des signaux numériques générés par le système d'acquisition et des signaux reçus du système central. Ladite interface peut être agencée entre le diviseur et les convertisseurs analogique numérique. On entend par modulation d'un signal numérique la transposition de ce signal numérique sur un signal de type porteuse pour transporter l'information de ce signal numérique.

Selon un exemple de réalisation de l'invention, l'unité de raccordement de chaque système d'acquisition comporte un coupleur agencé pour transmettre les signaux numériques convertis par les convertisseurs analogique numérique de ce système d'acquisition à l'unité de raccordement, le coupleur étant agencé pour isoler galvaniquement ces convertisseurs analogique numérique de l'unité de raccordement. Par exemple, le coupleur peut être un optocoupleur.

De façon alternative ou cumulative, chaque système d'acquisition comprend deux batteries électriques rechargeables et un système de commutation relié auxdites batteries électriques, à l'unité de puissance, aux amplificateurs opérationnels et aux convertisseurs analogique numérique de ce système d'acquisition, l'unité de puissance de ce système d'acquisition comprenant une unité de contrôle dudit système de commutation agencée pour simultanément :
a. connecter l'une des batteries électriques aux amplificateurs opérationnels et aux convertisseurs analogique numérique de ce système d'acquisition et déconnecter cette batterie électrique de l'unité de puissance ; et
b. Déconnecter l'autre des batteries électriques des amplificateurs opérationnels et des convertisseurs analogique numérique et connecter cette batterie électrique à l'unité de puissance.

Par exemple, l'unité de puissance peut comprendre un étage de puissance, notamment un convertisseur de puissance électrique, agencé pour recevoir ladite puissance électrique obtenue de la source d'énergie électrique et pour la convertir en une puissance électrique adaptée à la charge de la batterie électrique.

En d'autres termes, pour chaque système d'acquisition, l'unité de contrôle vient autoriser l'alimentation électrique de l'électronique active de ce système d'acquisition par l'une des batteries électriques et interdire la charge de cette batterie électrique au moyen de ladite puissance électrique obtenue de la source d'énergie électrique et simultanément interdire l'alimentation électrique de l'électronique active de ce système d'acquisition par l'autre des batteries électriques et autoriser la charge de cette batterie électrique au moyen de ladite puissance électrique obtenue de la source d'énergie électrique. Cette caractéristique permet de créer un isolement entre l'unité de puissance, dans laquelle les perturbations de la puissance électrique transitant sur le réseau de transmission de données sont présentes et l'électronique active du système d'acquisition, qui peut ainsi déterminer les différences de potentiels et les convertir en signaux numériques sans être perturbée.

Si on le souhaite, ledit potentiel électrique de référence est obtenu au moyen de la batterie électrique alimentant le dispositif d'acquisition. Le potentiel électrique de référence peut notamment être obtenu au moyen de la batterie électrique autorisée par le système de commutation à alimenter les amplificateurs opérationnels et les convertisseurs analogique numérique.

Par exemple, le système de commutation comporte un premier dispositif de commutation relié à l'unité de puissance et auxdites batteries électriques de sorte à connecter l'unité de puissance sélectivement à l'une ou l'autre des batteries et un deuxième dispositif de commutation relié auxdites batteries électriques et aux amplificateurs opérationnels et aux convertisseurs analogique numérique de sorte à connecter ces amplificateurs opérationnels et convertisseurs analogique numérique sélectivement à l'une ou l'autre des batteries, l'unité de contrôle étant agencée pour contrôler simultanément l'état des premier et deuxième dispositifs de commutation pour que ladite puissance électrique obtenue de la source d'énergie électrique soit transmise à l'une des batteries et que les amplificateurs opérationnels et convertisseurs analogique numérique soient alimentés par l'autre des batteries. En d'autres termes, le premier dispositif de commutation autorise la recharge exclusivement de l'une des batteries tandis que le deuxième dispositif de commutation autorise l'alimentation des amplificateurs opérationnels et convertisseurs analogique numérique exclusivement par l'autre des batteries.

Si on le souhaite, l'unité de puissance peut comporter une ou plusieurs capacités agencées pour délivrer une puissance électrique, lors de leur décharge, vers les amplificateurs opérationnels et convertisseurs analogique numérique, pendant les changements d'états des dispositifs de commutation.

Dans un mode de réalisation de l'invention, la source d'énergie électrique du système central comporte un panneau photovoltaïque.

Avantageusement, le système central comporte un régulateur de tension connecté au panneau photovoltaïque. Ce régulateur de tension est notamment agencé pour maintenir la puissance électrique, et notamment la tension électrique, fournie par le panneau photovoltaïque à une valeur constante. Selon un exemple de réalisation, le régulateur de tension peut être un régulateur de tension linéaire. Le cas échéant, le régulateur de tension peut être un régulateur linéaire à faible chute de tension. Un tel régulateur est également appelé régulateur LDO, de l'anglais Low DropOut. Dans un autre exemple de réalisation, le régulateur de tension peut être un régulateur de type DC/DC, par exemple un régulateur de type abaisseur de tension, notamment un régulateur de type Buck.

Dans un mode de réalisation de l'invention, le système central comporte une batterie électrique rechargeable, le panneau photovoltaïque étant agencé pour charger ladite batterie électrique, laquelle est ainsi apte à délivrer ladite puissance électrique aux systèmes d'acquisition. Selon une variante, le panneau photovoltaïque est agencé pour délivrer ladite puissance électrique aux systèmes d'acquisition.

Avantageusement, le panneau photovoltaïque est agencé pour simultanément charger la batterie électrique et délivrer ladite puissance électrique aux systèmes d'acquisition. Le cas échéant, le système peut comprendre une unité de contrôle de l'alimentation des systèmes d'acquisition, l'unité de contrôle étant agencée pour contrôler sélectivement l'alimentation des systèmes d'acquisition par le panneau photovoltaïque et/ou la batterie électrique, par exemple en fonction d'une condition de fonctionnement diurne ou nocturne du système d'analyse, d'une information d'ensoleillement local et/ou d'une information relative à l'état de charge de la batterie électrique.

Avantageusement, le système peut comporter un contrôleur de charge de ladite batterie électrique agencé pour réguler le courant électrique généré par le panneau photovoltaïque.

Avantageusement, la batterie électrique rechargeable présente une valeur de courant de charge nominale. Dans un premier mode de réalisation de l'invention, le contrôleur de charge est agencé pour contrôler la valeur du courant électrique généré par le panneau photovoltaïque pour que cette valeur corresponde sensiblement ladite valeur de courant de charge nominale. Par exemple, le contrôleur de charge peut être un hacheur. Dans ce mode, on cherche ainsi à recharger la batterie électrique le plus rapidement possible. Dans un deuxième mode de réalisation de l'invention, le contrôleur de charge est agencé pour contrôler la valeur du courant électrique généré par le panneau photovoltaïque à une valeur sensiblement inférieure à ladite valeur de courant de charge nominale, par exemple inférieure à 10%, notamment inférieure à 5%, par exemple égale à 2,2%, de ladite valeur de courant de charge nominale. Par exemple, le contrôleur de charge peut être une diode de limitation de courant. Dans ce mode, au-delà de la réduction du coût du système, on autorise une recharge lente de la batterie électrique, ce qui permet de réduire le bruit de cette recharge sur la mesure des signaux électriques émis par la ou les plantes par le premier dispositif d'acquisition.

Dans un mode de réalisation de l'invention, le système central comporte un module de raccordement relié, via ledit réseau de transmission de données, à chaque système d'acquisition, le module de raccordement étant apte à recevoir la puissance électrique fournie, directement ou indirectement, par le panneau photovoltaïque, notamment par l'intermédiaire du régulateur de tension, et à transmettre des signaux contenant ladite puissance électrique sur ledit réseau de données vers chaque système d'acquisition. De préférence, le module de raccordement est apte à recevoir les signaux numériques transmis par les unités de raccordement des systèmes d'acquisition sur le réseau de transmission de données.

Par exemple, le module de raccordement pourra être un commutateur réseau de type POE, pouvant être intégré dans un serveur informatique du système central apte à écrire et lire des données dans ladite mémoire, le commutateur étant relié au panneau photovoltaïque, notamment par l'intermédiaire du régulateur de tension. Le cas échéant, chaque système d'acquisition pourra être relié par un câble Ethernet, ou par une pluralité de câbles Ethernet reliés entre eux par un ou plusieurs répéteurs POE, à un port du commutateur réseau. En variante, le commutateur réseau pourra être déporté dudit serveur et relié audit serveur par un câble Ethernet.

En variante, le module de raccordement pourra comporter un ou plusieurs injecteur POE relié d'une part à un serveur informatique du système central apte à écrire et lire des données dans ladite mémoire et d'autre part au panneau photovoltaïque, notamment par l'intermédiaire du régulateur de tension. Le cas échéant, chaque injecteur POE pourra être relié à l'un des systèmes d'acquisition par un câble Ethernet. En variante, un injecteur POE pourra être relié à un collecteur relié à une pluralité de systèmes d'acquisition par une pluralité de câbles Ethernet.

Avantageusement, le système d'analyse comporte au moins un capteur, notamment une pluralité de capteurs, le ou chaque capteurs étant apte à mesurer un paramètre environnemental du champ, le ou chaque capteur étant relié au système central. Par exemple, le ou chaque capteur pourra être choisi parmi l'un des types de capteur suivants : capteur de la température de l'air ou du sol, capteur de l'humidité de l'air ou du sol, station météorologique, capteur de pression atmosphérique, anémomètre, luxmètre. Le cas échéant, le ou chaque capteur pourra être relié au système central en étant relié à un relai d'un système d'acquisition ou directement au système central.

Avantageusement, le système d'analyse électro-physiologique comporte un module de traitement numérique des signaux numériques générés par chaque système d'acquisition et transmis au système central pour y être stockés dans la mémoire.

Avantageusement, le module de traitement peut être agencé pour mettre en oeuvre un ou plusieurs procédés de traitement du signal numérique généré pour déterminer un état physiologique de la plante. Il a en effet été observé que les différences de potentiels ainsi mesurées et donc les signaux numériques convertis présentent des variations qui sont fonctions de l'état physiologique de la plante, et notamment de son ensoleillement, de son stress hydrique, de son stress thermique, etc. Ces variations sont des variations de certaines composantes fréquentielles de ces signaux numériques convertis, et en particulier des variations de faibles amplitudes de composantes haute fréquence.

Le module de traitement pourra être agencé pour procéder à une analyse spectrale des signaux numériques. Par exemple, le module de traitement pourra être agencé pour obtenir un spectre à partir de chaque signal numérique (par exemple au moyen d'une transformée de Fourier ou par une décomposition en ondelettes) et pour déterminer un ou plusieurs indicateurs de puissance spectrale à partir de ce spectre, et notamment un ou plusieurs indicateurs choisis parmi les indicateurs suivants :
a. un front de fréquence spectrale (également appelé en anglais : Spectral Edge Frequency ou SEF), à savoir une fréquence en dessous de laquelle se trouve concentré 95 % de la puissance spectrale totale ;
b. une fréquence médiane (également appelé en anglais : Médiane Edge Frequency ou MEF), à savoir une fréquence en dessous de laquelle se trouve concentré 50 % de la puissance spectrale totale;
c. un indice de puissance, à savoir la puissance spectrale totale contenue dans une bande de fréquence donnée, en notamment un indice de puissance dans les bandes de fréquence dite bêta (>0.5 Hz), alpha (0.3-0.4Hz), thêta (0.1-0.2 Hz) et delta (0-0.1 Hz);
d. des ratios d'indices de puissance, et notamment un ratio de l'indice de puissance delta et l'indice de puissance theta ;
e. une entropie spectrale (également appelée en en anglais Power Spectrum Entropy ou PSE).

Avantageusement, le module de traitement pourra mettre en oeuvre des procédés d'analyse des variations de ce ou ces indicateurs, notamment pour reconnaître un décalage du front de fréquence spectrale et/ou de la fréquence médiane selon un modèle donné ou encore une variation d'un indice de puissance ou d'un ratio d'indices de puissance selon un modèle donné, ou encore une variation de l'entropie spectrale selon un modèle donné. Il pourra par exemple s'agir d'un procédé de classification d'une variation de ce ou ces indicateurs parmi une bibliothèque de modèles de variation prédéterminés, chaque modèle de variation prédéterminé étant associé à un état physiologique donné d'une plante. Un tel procédé de classification pourra par exemple mis en oeuvre par un algorithme de reconnaissance de motifs basé un apprentissage automatique de reconnaissance des modèles de ladite bibliothèque de modèles.

Le module de traitement pourra être déporté du système central ou en variante être embarqué dans le système central, par exemple dans un serveur informatique du système central.

La présente invention est maintenant décrite à l'aide d'exemples uniquement illustratifs et nullement limitatifs de la portée de l'invention, et à partir des dessins annexés, dessins sur lesquels les différentes figures représentent :
[Fig. 1] représente, schématiquement et partiellement, un système d'analyse électro-physiologique de plantes selon un mode de réalisation de l'invention ;
[Fig. 2] représente, schématiquement et partiellement, un exemple de déploiement dans un champ du système d'analyse électro-physiologique de plantes de la [Fig. 1];
[Fig. 3] représente, schématiquement et partiellement, un exemple de réalisation du système central du système d'analyse électro-physiologique de plantes de la [Fig. 1];
[Fig. 4] représente, schématiquement et partiellement, un exemple de réalisation d'un relai du système d'analyse électro-physiologique de plantes de la [Fig. 1]; et
[Fig. 5] représente, schématiquement et partiellement, une vue schématique du relai de la [Fig. 4].

Dans la description qui suit, les éléments identiques, par structure ou par fonction, apparaissant sur différentes figures conservent, sauf précision contraire, les mêmes références.

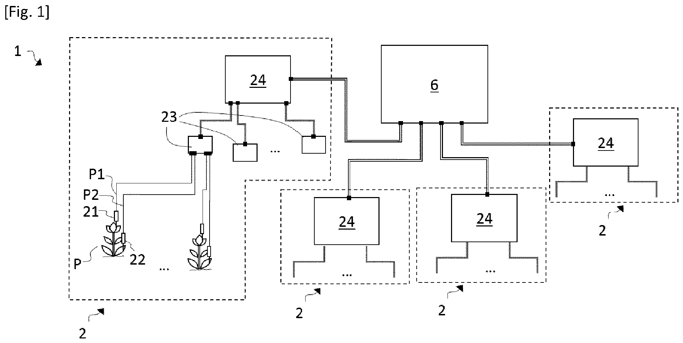

On a représenté en [Fig. 1], schématiquement et partiellement, un système d'analyse électro-physiologique 1 d'une pluralité de plantes P selon un mode de réalisation de l'invention.

Le système 1 est destiné à être déployé dans un champ agricole ou dans une serre pour y réaliser, de façon autonome et continue, des mesures de signaux électriques analogiques émis par des plantes cultivées dans ce champ ou dans cette serre.

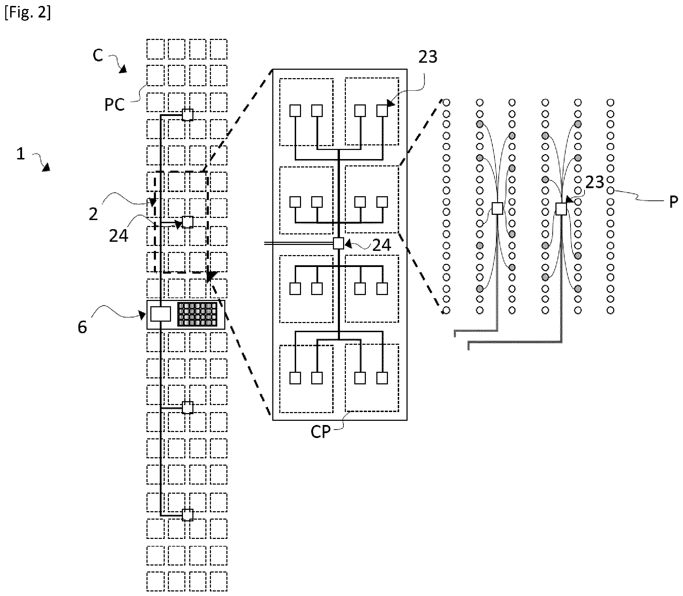

On a ainsi représenté en [Fig. 2] un exemple de déploiement du système 1 de la [Fig. 1] dans un champ C composé d'une pluralité de parcelles PC comportant chacune une pluralité de plantes P. Comme cela va être décrit, le système 1 permet de réaliser, de façon autonome, c'est-à-dire sans requérir à une source d'énergie externe au système 1 et de façon continue, pendant plusieurs jours voire pendant plusieurs semaines, des mesures sur plusieurs centaines de ces plantes P, réparties dans différentes parcelles PC s'étendant sur une vaste surface du champ C.

Le système 1 comporte une pluralité de systèmes d'acquisition 2 reliés chacun à un même système central 6. Dans l'exemple de la [Fig. 2], le système 1 comporte quatre systèmes d'acquisition 2, tous semblables les uns aux autres.

Chaque système d'acquisition 2 comporte une pluralité de paires de premières électrodes 21 et de deuxièmes électrodes 22, chaque paire étant connectée à l'une des plantes P. Par exemple, la première électrode 21 est connectée au niveau d'une section supérieure de tige d'une plante P pour être en contact avec le phloème et la deuxième électrode 22 est connectée au niveau d'une section inférieure de la tige de cette plante P également pour être en contact avec le phloème. Dans l'exemple de la [Fig. 2], chaque système d'acquisition 2 comporte 128 paires d'électrodes 21 et 22.

Chaque première électrode 21 capte un premier potentiel électrique analogique P1 et chaque deuxième électrode 22 capte un deuxième potentiel électrique analogique P2.

Chaque système d'acquisition 2 comporte une pluralité de terminaux 23 relié chacun à un groupe de paires d'électrodes 21 et 22. Dans l'exemple de la [Fig. 2], chaque système d'acquisition 2 comporte seize terminaux 23, réalisés chacun sous la forme d'un bornier auquel sont reliés huit paires d'électrodes 21 et 22, le bornier collectant ainsi les premiers et deuxièmes potentiels électriques analogiques P1 et P2 captés par ces huit électrodes.

Chaque système d'acquisition 2 comporte un relai 24 relié aux différents terminaux 23. Comme cela sera décrit ultérieurement, chaque relai 24 comporte une pluralité d'amplificateurs opérationnels destinés à mesurer les différences de potentiels entre les premiers et deuxièmes potentiels électriques analogiques P1 et P2 captés par chaque paire d'électrodes 21 et 22 de ce système d'acquisition 2, ainsi qu'une pluralité de convertisseurs analogique numérique destinés à convertir ces différences de potentiel en des signaux numériques.

Chaque relai 24 comporte en outre une unité de raccordement à un réseau de transmission de données pour être relié au système central 6 et pour pouvoir transmettre à ce système central 6 lesdits signaux numériques. On constate ainsi, au vu de la [Fig. 2], que le système d'analyse 1 est un réseau de capteurs formés par les plantes P, présentant une topologie en étoile autour du système central 6, chaque système d'acquisition 1 présentant une topologie en arbre dont la racine est formée par le relai 24 et dont les terminaisons sont formées par les plantes P. On notera que le nombre de relais 24 et de terminaux 23 décrits en [Fig. 2] est exemplatif et qu'on pourra envisager un nombre différent, et notamment supérieur, de relais et de terminaux sans sortir du cadre de la présente invention. De même, on pourra envisager d'ajouter des concentrateurs entre chaque relai 24 et les terminaux 23, afin de d'accroitre le nombre de points de mesure dans une même zone du champ C, ou encore d'ajouter des extenseurs afin d'atteindre des parcelles éloignées du système central 6.

Comme cela sera décrit ultérieurement, le système central 6 comporte un serveur informatique embarquant une mémoire dans laquelle sont stockés les différents signaux numériques transmis par les relais 24, lesquels peuvent alors être traités in situ ou au contraire réservés pour un traitement ultérieur. Par ailleurs, afin d'obtenir un système d'analyse pouvant réaliser des mesures de façon autonome et continue pendant une longue période, le système central 6 comporte une source d'énergie électrique 8, permettant de fournir une puissance électrique aux amplificateurs opérationnels et aux convertisseurs analogique numérique des relais 24. Dans l'exemple de la [Fig. 2], la source d'énergie électrique 8 comporte un panneau photovoltaïque. Chaque relai 24 comporte ainsi une unité de puissance destinée à recevoir ladite puissance électrique transmis par le système central 6 et à transmettre cette puissance électriques aux amplificateurs opérationnels et aux convertisseurs analogique numérique de ce relai 24.

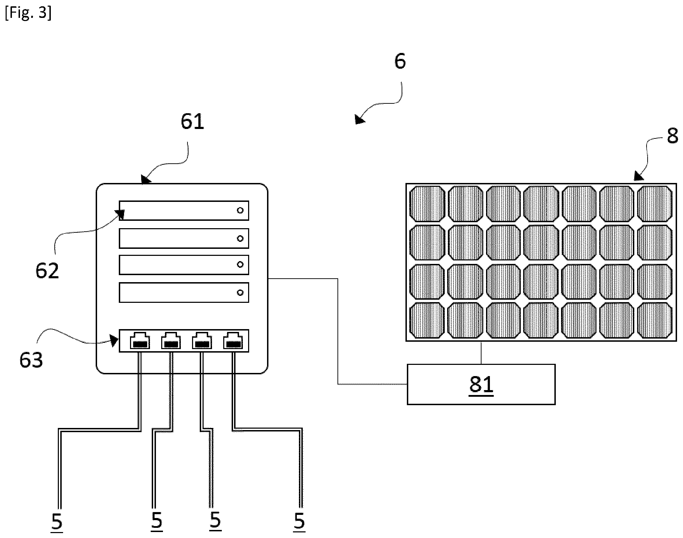

En lien avec la [Fig. 3], on va maintenant décrire un mode de réalisation préférentiel du système central 6 des [Fig. 1] et [Fig. 2].

Comme décrit précédemment, le système central 6 comporte un serveur informatique 61, de type stockage en réseau, ou NAS, basse consommation, accueillant une pluralité de disques dur 62 formant ladite mémoire du système central 6. Le système 1 également comporte un panneau photovoltaïque 8 associé à un régulateur de tension linéaire 81 de type LDO, régulant la puissance électrique générée par le panneau photovoltaïque pour fournir une tension électrique de valeur constante au serveur 61. On pourra envisager d'ajouter au système central 6 une ou plusieurs batteries électriques, reliées d'une part au serveur 61, via une unité de commutation, et d'autre part, au régulateur 81, via un régulateur de charge. Dans ce cas, une unité de contrôle pourra contrôler l'unité de commutation et le contrôleur de charge de sorte à autoriser, en période diurne, l'alimentation du serveur 61 directement par le panneau photovoltaïque 8 et la charge de la ou des batteries électriques, l'alimentation 61 du serveur par les batteries électriques étant dans ce cas interdit, et de sorte à autoriser, en période nocturne, l'alimentation du serveur 61 par les batteries électriques .

Le serveur 61 embarque également un commutateur réseau 63 de type POE pour échanger des données avec les relais 24 des systèmes d'acquisition 2, et en particulier pour recevoir les signaux numériques transmis par ces relais 24, et pour pouvoir transmettre la puissance électrique générée par le panneau photovoltaïque 8 et le régulateur de tension 81 aux amplificateurs opérationnels et aux convertisseurs analogique numérique de ces relais 24.

Le relai 24 de chaque système d'acquisition 2 est ainsi relié à un port de ce commutateur réseau 63 par un câble Ethernet 5, c'est-à-dire un câble comportant quatre paires de fils torsadées, les données transmises par les relais 24 ou par le serveur 61 transitant sur deux des paires et la puissance électrique transitant sur deux autres paires.

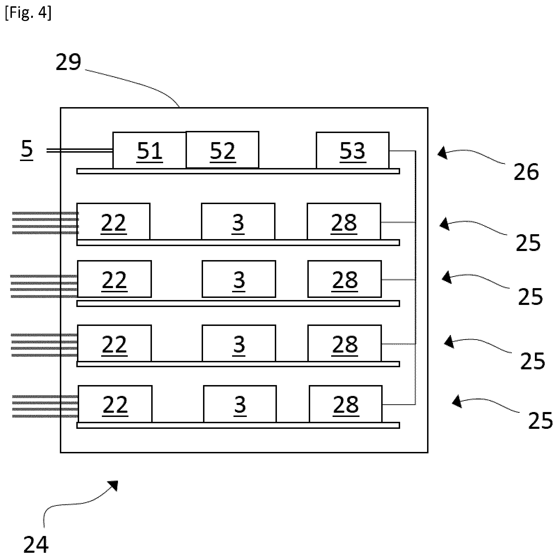

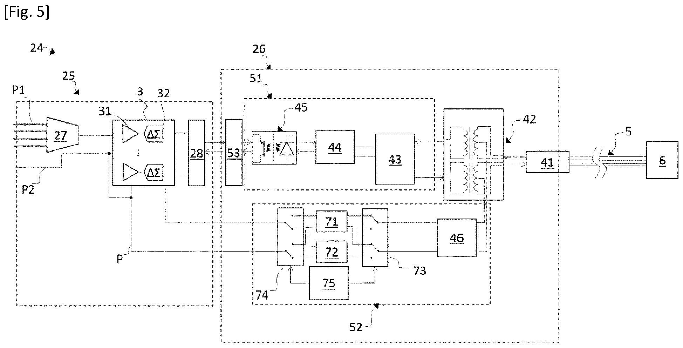

En lien avec les [Fig. 4] et [Fig. 5], on va maintenant décrire un mode de réalisation préférentiel d'un relai 24 d'un système d'acquisition 2 destiné à être relié au système central 6 de la [Fig. 3].

Le relai 24 comporte plusieurs étages d'acquisition 25 destinés à recevoir les potentiels électriques analogiques P1 et P2 transmis par les terminaux 23 et comportant chacun une pluralité d'amplificateurs opérationnels et de convertisseurs analogique numérique pour procéder à la mesure des différences de potentiel et à la conversion de ces différences de potentiel en signaux numériques et un étage de raccordement 26 auquel sont reliés tous ces étages d'acquisition 25. Cet étage de raccordement 26 comporte ladite unité de raccordement 51 permettant de transmettre ces signaux numériques au système central 6 via le câble Ethernet 5 et ladite unité de puissance 52 permettant d'extraire de ce câble Ethernet ladite puissance électrique transmise par le système central 6 pour la distribuer aux amplificateurs opérationnels et aux convertisseurs analogique numérique des étages d'acquisition 25. La [Fig. 4] montre ainsi une vue d'ensemble d'un relai 24 tandis que la [Fig. 5] décrit de façon schématique l'un des étages d'acquisition 25 et l'étage de raccordement 26.

Dans l'exemple des [Fig. 4] et [Fig. 5], chaque étage d'acquisition 25 est relié, par plusieurs câbles coaxiaux multiples, à quatre terminaux 23, pour recevoir de chacun de ces terminaux, huit paires de potentiels électriques analogiques P1 et P2. On notera que, dans l'exemple décrit, chaque deuxième électrode 22 est reliée à un potentiel électrique commun de référence PR, et notamment une masse, de l'étage d'acquisition 25 auquel cette deuxième électrode 22 est reliée, de sorte que chaque deuxième potentiel électrique analogique P2 soit sensiblement égal à ce potentiel électrique commun de référence PR.

Chaque étage d'acquisition 25 comporte un multiplexeur multivoies 27, dont les entrées sont reliées, via les terminaux 23, aux premières électrodes 21. Le multiplexeur 27 est ainsi agencé pour multiplexer les premiers potentiels électriques P1 captés par les premières électrodes 21 et transmis par les terminaux 23. Chaque étage d'acquisition 25 traite donc de façon séquentielle les premiers potentiels électriques P1 transmis par les terminaux 23, l'ensemble des premiers potentiels électriques P1 transmis par chaque terminal 23 étant traités sur une même période de multiplexage.

Chaque étage d'acquisition 25 comporte également un convertisseur analogique numérique multivoies 3, comportant une pluralité d'amplificateurs opérationnels 31 chacun agencés pour recevoir l'une des sorties du multiplexeur 27, en l'occurrence un premier potentiel électrique analogique P1 acquis par l'une des premières électrodes 21 et transmis par l'un des terminaux 23, et ledit potentiel électrique commun de référence PR et pour mesurer une différence de potentiel électrique entre ce premier potentiel P1 et le potentiel électrique commun de référence PR. Dans l'exemple décrit, chaque amplificateur opérationnel 31 est un amplificateur à gain programmable, également appelé PGA (de l'anglais Programmable Gain Amplifier), à faible bruit et dont le gain peut être programmé jusqu'à une valeur de 24 sans qu'un niveau moyen du bruit introduit dans le signal amplifié par l'amplificateur dépasse une valeur de sensiblement 2 µV pour ce gain de 24.

Le convertisseur analogique numérique multivoies 3 comporte également une pluralité de convertisseurs analogique numérique élémentaire 32 de type delta-sigma chacun relié à la sortie différentielle de l'un des amplificateurs opérationnels 31 pour convertir la différence de potentiel électrique mesuré par cet amplificateur opérationnel 31 en un signal numérique. Dans l'exemple décrit, chaque convertisseur de type delta-sigma 34 est agencé pour échantillonner la différence de potentiel électrique, par exemple à une fréquence d'échantillonnage de 250 Hz, de sorte à obtenir un signal numérique codé sur 24 bits. Cette combinaison de PGA à faible bruit et de convertisseurs de type delta-sigma permet d'obtenir un nombre de bits effectifs d'au moins 19 lorsque le gain de PGA est fixé à 24. De la sorte, le signal numérique obtenu en sortie du convertisseur élémentaire 32 présente une résolution particulièrement satisfaisante, compte tenu des niveaux de tension observés pour les premiers potentiels électriques captés par les électrodes 21. Il est à noter que la fréquence d'échantillonnage peut avantageusement être modulée, notamment en fonction de la durée d'enregistrement souhaitée.

Enfin, chaque étage d'acquisition 25 comporte un microcontrôleur 28 agencé pour sérialiser les signaux numériques convertis par chacun des convertisseurs élémentaires 32, de sorte que ce système d'acquisition 25 soit relié à l'étage de raccordement 26 par une voie unique sur laquelle transitent ces signaux numériques sérialisés. De même, l'étage de raccordement 26 comporte un microcontrôleur 53 agencé pour sérialiser les signaux numériques transmis par tous les étages d'acquisition 25. Dans l'exemple décrit, l'ensemble des composants de chaque étage d'acquisition 25 sont supportés par une même carte de circuit imprimé, les étages d'acquisition 25 et l'étage de raccordement étant ainsi empilés et logés dans un même boîtier 29. Tous ces éléments forment ainsi un ensemble compact et autonome.

Comme on va le voir, l'étage de raccordement 26 de chaque relai 24 est agencé pour isoler galvaniquement les étages d'acquisition 25 des unités de raccordement et de puissance 51 et 52.

Dans l'exemple décrit, l'étage de raccordement 26 comporte un connecteur de type RJ45 41 auquel est raccordé le câble Ethernet 5 le reliant au système central 6.

Afin d'isoler galvaniquement les différents composants de l'étage de raccordement 26 des autres relais 24 et des autres capteurs pouvant être connectés au système central 6 et d'extraire ladite puissance électrique transmise par le système central 6 via le câble Ethernet 5, l'étage de raccordement 26 comporte un diviseur 42 comportant une pluralité de transformateurs d'isolement chacun relié, via le connecteur 41, à l'une des paires torsadées au niveau de leur enroulement primaire ou secondaire. En [Fig. 5], seuls les transformateurs reliés aux paires torsadées transportant la puissance électrique ont été représentés. Le diviseur 42 est ainsi agencé pour extraire la puissance électrique transitant sur le câble 5 depuis les enroulements de ces transformateurs.

L'unité de raccordement 51 comporte une interface 43 permettant de convertir les signaux reçus depuis le câble 5 et le diviseur 42 en données numériques interprétables et, réciproquement, les signaux numériques issus du sérialiseur 53 en des signaux pouvant être transmis, via le diviseur 42 et le connecteur 41, sur le câble 5.

L'unité de raccordement 51 comporte une unité de contrôle 44 reliée à l'interface 43 agencée notamment pour traiter les signaux reçus depuis le câble 5.

L'unité de raccordement 51 comporte un optocoupleur agencé pourtransmettre les signaux numériques issus du sérialiseur 53 à l'unité de contrôle 44, tout en maintenant un isolement galvanique entre l'unité de contrôle 44 et ce sérialiseur 53 et donc les étages d'acquisition 25.

L'unité de puissance 52 comporte deux batteries électriques rechargeables 71 et 72 agencées pour, d'une part, alimenter électriquement les étages d'acquisition 25, et notamment les convertisseurs analogique numérique multivoies 3, et d'autre part, définir le potentiel électrique de référence P.

L'unité de puissance 52 comporte un convertisseur de puissance électrique 46 agencé pour recevoir la puissance électrique extraite depuis le diviseur 42 et la convertir en une puissance électrique adaptée pour la charge des batteries électriques 71 et 72.

Les batteries 71 et 72 sont ainsi reliées, d'une part, au convertisseur de puissance 46 et, d'autre part, aux étages d'acquisition par deux dispositifs de commutation 73 et 74 contrôlées simultanément par une unité de contrôle 75 de l'unité de puissance 52.

L'unité de contrôle 75 contrôle ainsi le premier dispositif de commutation 73 de sorte à autoriser uniquement la transmission de la puissance d'alimentation convertie par le convertisseur de puissance 46 vers l'une des batteries électriques, la batterie 71 dans l'exemple de la [Fig. 5], en vue de sa charge. Dans le même temps, l'unité de contrôle 75 contrôle le deuxième dispositif de commutation 74 de sorte à autoriser uniquement la transmission de la puissance électrique contenue dans l'autre des batteries électriques, la batterie 72 dans l'exemple de la [Fig. 5], vers les étages d'acquisition 25, en vue de leurs alimentations électriques et de la fourniture du potentiel électrique de référence P2. En effet, les entrées du convertisseur analogique numérique 3 destinées à recevoir les deuxièmes potentiels électriques analogiques sont reliées, via le deuxième dispositif de commutation 74, à la masse de cette batterie 72 alimentant ces étages d'acquisition 25.

En d'autres termes, l'unité de contrôle 75 contrôle les dispositifs de commutation 73 et 74 de sorte qu'il soit impossible à la batterie 71 en charge d'alimenter électriquement les étages d'acquisition 25 et qu'il soit impossible à la batterie 72 alimentant les étages d'acquisition 25 et fournissant le potentiel de référence P2 d'être chargée par le convertisseur de puissance 46.

On constate ainsi au vu de la [Fig. 5] que, grâce aux dispositifs de commutation 73 et 74 et à l'optocoupleur 45, le potentiel de référence P2 permettant la génération des signaux numériques est indépendant de toute perturbation pouvant intervenir sur le câble Ethernet 5 et en particulier de toute perturbation de la masse de l'alimentation électrique transitant sur ce câble.

Il est à relever que le système d'analyse 1 peut comporter un module de traitement numérique des signaux numériques stockés dans la mémoire du serveur 61, lequel est agencé pour mettre en oeuvre un ou plusieurs procédés de traitement de ces signaux numériques afin de déterminer un état physiologique des plantes P. Sans que cela soit limitatif, le module de traitement pourra être agencé pour obtenir un spectre à partir de chaque signal numérique (par exemple au moyen d'une transformée de Fourier ou par une décomposition en ondelettes) et pour déterminer un ou plusieurs indicateurs de puissance spectrale à partir de ces spectres, puis pour mettre en oeuvre des procédés d'analyse des variations de ce ou ces indicateurs afin de déterminer l'état physiologique des plantes P. Ce module de traitement numérique pourra être embarqué dans le serveur 61 ou être distant du serveur 61.

La description qui précède explique clairement comment l'invention permet d'atteindre les objectifs qu'elle s'est fixée, et notamment en proposant un système d'analyse électro-physiologique de plantes comportant une pluralité de points de mesure organisés selon une architecture en étoile autour d'un système central.

En tout état de cause, l'invention ne saurait se limiter aux modes de réalisation spécifiquement décrits dans ce document, et s'étend en particulier à tous moyens équivalents et à toute combinaison techniquement opérante de ces moyens. On pourrait notamment prévoir d'agencer les amplificateurs opérationnels et/ou les convertisseurs analogique numérique au niveau des terminaux des systèmes d'acquisition, de sorte à rendre négligeable les pertes induites par la longueur de l'ensemble des câbles reliant les électrodes au système central. On pourrait également envisager d'autres modes de réalisation permettant de transmettre la puissance électrique du panneau photovoltaïque aux relais des systèmes d'acquisition, notamment mettant en oeuvre des concentrateurs et/ou des injecteurs POE déportés du serveur du système central. On pourrait également envisager de remplacer l'un ou plusieurs des terminaux et/ou des relais par un capteur apte à mesurer un paramètre environnemental du camp. On pourra encore envisager de ne pas relier les deuxièmes électrodes à un même potentiel électrique de référence commun et d'ajouter un second multiplexeur aux étages d'acquisition permettant de multiplexer les deuxièmes potentiels électriques analogiques captés par ces deuxièmes électrodes.

## Revendications

1. Système d'analyse électro-physiologique de plantes, **caractérisé en ce qu'**il comporte :
a. Une pluralité de systèmes d'acquisition, chaque système d'acquisition comportant :
i. une pluralité de paires d'électrodes comportant chacune une première électrode et d'une deuxième électrode destinées à être connectée chacune à une même plante pour capter des premier et deuxième potentiels électriques analogiques;
ii. une pluralité d'amplificateurs opérationnels agencés pour mesurer les différences de potentiel entre les premiers et deuxièmes potentiels électriques analogiques acquis par les premières et deuxièmes électrodes de ce système d'acquisition et une pluralité de convertisseurs analogique numérique agencés pour convertir lesdites différences de potentiel en des signaux numériques ;
b. Un système central de collecte de données comportant une mémoire et une source d'énergie électrique ;
**caractérisé en ce que** chaque système d'acquisition est relié au système central et comporte :
c. une unité de raccordement agencée pour transmettre lesdits signaux numériques au système central ;
d. une unité de puissance agencée pour transmettre une puissance électrique obtenue de ladite source d'énergie électrique aux amplificateurs opérationnels et aux convertisseurs analogiques numériques ; et
dans lequel chaque système d'acquisition est agencé pour isoler galvaniquement les amplificateurs opérationnels et les convertisseurs analogique numérique de l'unité de puissance et de l'unité de raccordement.

2. Système selon la revendication précédente, dans lequel chaque système d'acquisition comporte une pluralité de terminaux reliés chacun à un groupe de paires d'électrodes de ce système d'acquisition et agencés pour collecter lesdites potentiels électriques analogiques captés par les premières et deuxièmes électrodes de ce groupe, et un relai relié auxdits terminaux de ce système d'acquisition et au système central, ce relai comportant ladite unité de raccordement et ladite unité de puissance, dans lequel les amplificateurs opérationnels et les convertisseurs analogique numérique de chaque système d'acquisition sont prévus dans le relai et/ou dans les terminaux de ce système d'acquisition, dans lequel le relai de chaque système d'acquisition est agencé pour isoler galvaniquement ces amplificateurs opérationnels et ces convertisseurs analogique numérique de ce système d'acquisition de son unité de puissance et de son unité de raccordement,.

3. Système selon la revendication précédente, dans lequel chaque terminal de chaque système d'acquisition est agencé pour transmettre les potentiels électriques analogiques qu'il collecte vers le relai de ce système d'acquisition, dans lequel le relai de chaque système d'acquisition comporte une pluralité d'étages d'acquisition chacun relié au moins à l'un des terminaux de ce système d'acquisition pour recevoir lesdits potentiels électriques analogiques collectés et comportant au moins un amplificateur opérationnel et un convertisseur analogique numérique pour mesurer lesdites différences de potentiel et convertir lesdites différences de potentiel en lesdits signaux numériques, chaque étage d'acquisition étant relié à l'unité de raccordement et l'unité de puissance en étant isolé galvaniquement de cette unité de puissance et de son unité de raccordement.

4. Système selon la revendication précédente, dans lequel chaque étage d'acquisition du relai de chaque système d'acquisition est relié à une pluralité de terminaux de ce système d'acquisition pour recevoir lesdits potentiels électriques analogiques collectés par ces terminaux, ledit étage comportant un multiplexeur agencé pour multiplexer lesdits premiers potentiels électriques analogiques collectés par lesdits terminaux.

5. Système selon la revendication 2, dans lequel chaque terminal de chaque système d'acquisition comporte au moins un amplificateur opérationnel pour mesurer lesdites différences de potentiel entre les potentiels électriques analogiques qu'il collecte et pour transmettre ces différences de potentiel vers le relai de ce système d'acquisition, et dans lequel le relai de chaque système d'acquisition comporte une pluralité d'étages d'acquisition chacun relié au moins à l'un des terminaux de ce système d'acquisition pour recevoir lesdits différences de potentiels électriques analogiques, chaque étage d'acquisition étant relié à l'unité de raccordement et l'unité de puissance en étant isolé galvaniquement de cette unité de puissance et de son unité de raccordement.

6. Système selon l'une des revendications 2 à 5, dans lequel l'unité de raccordement comporte un microcontrôleur agencé pour sérialiser les signaux numériques convertis par les convertisseurs analogique numérique et transmis par chacun des étages d'acquisition à l'unité de raccordement en un unique signal numérique et pour transmettre cet unique signal numérique au système central.

7. Système selon l'une des revendications précédentes, dans lequel le système central est relié à chaque système d'acquisition par un réseau de transmission de données, dans lequel le système central est agencé pour transmettre à chaque système d'acquisition des signaux contenant une puissance électrique via le réseau de transmission de données, et, dans lequel l'unité de raccordement est agencée pour transmettre lesdits signaux numériques au système central via ledit réseau de transmission de données, et dans lequel l'unité de puissance est agencée pour extraire ladite puissance électrique depuis les signaux reçus du système central via le réseau de transmission de donnée et pour transmettre ladite puissance électrique extraite aux amplificateurs opérationnels et aux convertisseurs analogiques numériques.

8. Système selon l'une des revendications précédentes, dans lequel l'unité de raccordement de chaque système d'acquisition comporte un coupleur agencé pour transmettre les signaux numériques convertis par les convertisseurs analogique numérique de ce système d'acquisition à l'unité de raccordement, le coupleur étant agencé pour isoler galvaniquement ces convertisseurs analogique numérique de l'unité de raccordement.

9. Système selon l'une des revendications précédentes, dans lequel chaque système d'acquisition comprend deux batteries électriques rechargeables et un système de commutation relié auxdites batteries électriques, à l'unité de puissance, aux amplificateurs opérationnels et aux convertisseurs analogique numérique de ce système d'acquisition, l'unité de puissance de ce système d'acquisition comprenant une unité de contrôle dudit système de commutation agencée pour simultanément :
• connecter l'une des batteries électriques aux amplificateurs opérationnels et aux convertisseurs analogique numérique de ce système d'acquisition et déconnecter cette batterie électrique de l'unité de puissance ; et
• Déconnecter l'autre des batteries électriques des amplificateurs opérationnels et des convertisseurs analogique numérique et connecter cette batterie électrique à l'unité de puissance.

10. Système selon l'une des revendications précédentes, dans lequel la source d'énergie électrique du système central comporte un panneau photovoltaïque.

## Patentansprüche

1. System für eine elektrophysiologische Analyse von Pflanzen,
**dadurch gekennzeichnet, dass** es umfasst:
a. eine Vielzahl von Erfassungssystemen, jedes Erfassungssystem umfassend:
i. eine Vielzahl von Elektrodenpaaren, die jeweils eine erste Elektrode und eine zweite Elektrode umfassen, die dafür bestimmt sind, jeweils mit einer gleichen Pflanze verbunden zu werden, um ein erstes und ein zweites analoges elektrisches Potenzial zu empfangen;
ii. eine Vielzahl von Operationsverstärkern, die angeordnet sind, um die Potenzialdifferenzen zwischen dem ersten und dem zweiten analogen elektrischen Potenzial zu messen, die durch die erste und die zweite Elektrode dieses Erfassungssystems erfasst werden, und eine Vielzahl von Analog-Digital-Wandlern, die angeordnet sind, um die Potenzialdifferenzen in digitale Signale umzuwandeln;
b. ein Zentralsystem zum Sammeln von Daten, umfassend einen Speicher und eine elektrische Energiequelle;
**dadurch gekennzeichnet, dass** jedes Erfassungssystem mit dem Zentralsystem verknüpft ist und umfasst:
c. eine Anschlusseinheit, die angeordnet ist, um die digitalen Signale an das Zentralsystem zu übertragen;
d. eine Leistungseinheit, die angeordnet ist, um eine von der elektrischen Energiequelle erhaltene elektrische Leistung an die Operationsverstärker und an die Analog-Digital-Wandler zu übertragen; und
wobei jedes Erfassungssystem angeordnet ist, um die Operationsverstärker und die Analog-Digital-Wandler von der Leistungseinheit und der Anschlusseinheit galvanisch zu isolieren.

2. System nach dem vorstehenden Anspruch, wobei jedes Erfassungssystem eine Vielzahl von Endgeräten, die jeweils mit einer Gruppe von Elektrodenpaaren dieses Erfassungssystems verknüpft und angeordnet sind, um die analogen elektrischen Potenziale zu sammeln, die durch die erste und die zweite Elektrode dieser Gruppe empfangen werden, und ein Relais umfasst, das mit den Endgeräten dieses Erfassungssystems und mit dem Zentralsystem verknüpft ist, wobei dieses Relais die Anschlusseinheit und die Leistungseinheit umfasst, wobei die Operationsverstärker und die Analog-Digital-Wandler jedes Erfassungssystems in dem Relais und/oder in den Endgeräten dieses Erfassungssystems vorgesehen sind, wobei das Relais jedes Erfassungssystems angeordnet ist, um diese Operationsverstärker und diese Analog-Digital-Wandler dieses Erfassungssystems von seiner Leistungseinheit und von seiner Anschlusseinheit galvanisch zu isolieren.

3. System nach dem vorstehenden Anspruch, wobei jedes Endgerät jedes Erfassungssystems angeordnet ist, um die analogen elektrischen Potenziale, die es sammelt, an das Relais dieses Erfassungssystems zu übertragen, wobei das Relais jedes Erfassungssystems eine Vielzahl von Erfassungsstufen umfasst, die jeweils mit mindestens einem der Endgeräte dieses Erfassungssystems verknüpft sind, um die gesammelten analogen elektrischen Potenziale aufzunehmen, und umfassend mindestens einen Operationsverstärker und einen Analog-Digital-Wandler, um die Potenzialdifferenzen zu messen und die Potenzialdifferenzen in die digitalen Signale umzuwandeln, wobei jede Erfassungsstufe mit der Anschlusseinheit und der Leistungseinheit verknüpft ist, indem sie von dieser Leistungseinheit und ihrer Anschlusseinheit galvanisch getrennt ist.

4. System nach dem vorstehenden Anspruch, wobei jede Erfassungsstufe des Relais jedes Erfassungssystems mit einer Vielzahl von Endgeräten dieses Erfassungssystems verknüpft ist, um die analogen elektrischen Potenziale, die durch diese Endgeräte gesammelt werden, aufzunehmen, die Stufe umfassend einen Multiplexer, der angeordnet ist, um die ersten analogen elektrischen Potenziale, die durch die Endgeräte gesammelt werden, zu multiplexen.

5. System nach Anspruch 2, wobei jedes Endgerät jedes Erfassungssystems mindestens einen Operationsverstärker umfasst, um die Potenzialdifferenzen zwischen den analogen elektrischen Potenzialen, die es sammelt, zu messen und um diese Potenzialdifferenzen an das Relais dieses Erfassungssystems zu übertragen, und wobei das Relais jedes Erfassungssystems eine Vielzahl von Erfassungsstufen umfasst, die jeweils mit mindestens einem der Endgeräte dieses Erfassungssystems verknüpft sind, um die analogen elektrischen Potenzialdifferenzen aufzunehmen, wobei jede Erfassungsstufe mit der Anschlusseinheit und der Leistungseinheit verknüpft ist, indem sie von dieser Leistungseinheit und ihrer Anschlusseinheit galvanisch isoliert ist.

6. System nach einem der Ansprüche 2 bis 5, wobei die Anschlusseinheit einen Mikrocontroller umfasst, der angeordnet ist, um die digitalen Signale, die durch die Analog-Digital-Wandler umgewandelt und durch jede der Erfassungsstufen an die Anschlusseinheit übertragen werden, zu einem eindeutigen digitalen Signal zu serialisieren und um dieses eindeutige digitale Signal an das Zentralsystem zu übertragen.

7. System nach einem der vorstehenden Ansprüche, wobei das Zentralsystem mit jedem Erfassungssystem durch ein Datenübertragungsnetz verknüpft ist, wobei das Zentralsystem angeordnet ist, um über das Datenübertragungsnetz Signale, die eine elektrische Leistung enthalten, an jedes Erfassungssystem zu übertragen, und wobei die Anschlusseinheit angeordnet ist, um die digitalen Signale über das Datenübertragungsnetz an das Zentralsystem zu übertragen, und wobei die Leistungseinheit angeordnet ist, um die elektrische Leistung von den von dem Zentralsystem über das Datenübertragungsnetz aufgenommenen Signalen zu extrahieren und um die extrahierte elektrische Leistung an die Operationsverstärker und an die Analog-Digital-Wandler zu übertragen.

8. System nach einem der vorstehenden Ansprüche, wobei die Anschlusseinheit jedes Erfassungssystems einen Koppler umfasst, der angeordnet ist, um die durch die Analog-Digital-Wandler dieses Erfassungssystems umgewandelten digitalen Signale an die Anschlusseinheit zu übertragen, wobei der Koppler angeordnet ist, um diese Analog-Digital-Wandler von der Anschlusseinheit galvanisch zu isolieren.

9. System nach einem der vorstehenden Ansprüche, wobei jedes Erfassungssystem zwei aufladbare elektrische Batterien und ein Schaltsystem aufweist, das mit den elektrischen Batterien, mit der Leistungseinheit, mit den Operationsverstärkern und mit den Analog-Digital-Wandlern dieses Erfassungssystems verknüpft ist, wobei die Leistungseinheit dieses Erfassungssystems eine Steuereinheit des Schaltsystems aufweist, die angeordnet ist, um gleichzeitig:
• eine der elektrischen Batterien mit den Operationsverstärkern und mit den Analog-Digital-Wandlern dieses Erfassungssystems zu verbinden und diese elektrische Batterie von der Leistungseinheit zu trennen; und
• die andere der elektrischen Batterien von den Operationsverstärkern und den Analog-Digital-Wandlern zu trennen und diese elektrischen Batterie mit der Leistungseinheit zu verbinden.

10. System nach einem der vorstehenden Ansprüche, wobei die elektrische Energiequelle des Zentralsystems ein Photovoltaikpanel umfasst.

## Claims

1. A system for electro-physiological analysis of plants,
**characterized in that** it comprises:
a. a plurality of acquisition systems, each acquisition system comprising:
i. a plurality of pairs of electrodes each comprising a first electrode and a second electrode intended to each be connected to the same plant in order to pick up first and second analog electrical potentials;
ii. a plurality of operational amplifiers arranged to measure the differences in potential between the first and second analog electrical potentials acquired by the first and second electrodes of this acquisition system, and a plurality of analog-to-digital converters arranged to convert the differences in potential into digital signals;
b. a central data collection system comprising a memory and an electrical energy source;
**characterized in that** each acquisition system is connected to the central system and comprises:
c. a connection unit arranged to transmit the digital signals to the central system;
d. a power unit arranged to transmit electrical power obtained from the electrical energy source to the operational amplifiers and to the analog-to-digital converters; and
wherein each acquisition system is arranged to galvanically isolate the operational amplifiers and the analog-to-digital converters from the power unit and from the connection unit.

2. The system according to the preceding claim, wherein each acquisition system comprises a plurality of terminals each connected to a group of electrode pairs of this acquisition system and arranged to collect the analog electrical potentials picked up by the first and second electrodes of this group, and a relay connected to the terminals of this acquisition system and to the central system, this relay comprising said connection unit and said power unit, wherein the operational amplifiers and the analog-to-digital converters of each acquisition system are provided in the relay and/or in the terminals of this acquisition system, wherein the relay of each acquisition system is arranged to galvanically isolate these operational amplifiers and these analog-to-digital converters of this acquisition system from its power unit and from its connection unit.

3. The system according to the preceding claim, wherein each terminal of each acquisition system is arranged to transmit the analog electrical potentials that it collects to the relay of this acquisition system, wherein the relay of each acquisition system comprises a plurality of acquisition stages each connected at least to one of the terminals of this acquisition system in order to receive the collected analog electrical potentials, and comprising at least one operational amplifier and one analog-to-digital converter in order to measure the differences in potential and convert the differences in potential into the digital signals, each acquisition stage being connected to the connection unit and the power unit while being galvanically isolated from this power unit and from its connection unit.

4. The system according to the preceding claim, wherein each acquisition stage of the relay of each acquisition system is connected to a plurality of terminals of this acquisition system in order to receive the analog electrical potentials collected by these terminals, said stage comprising a multiplexer arranged to multiplex said first analog electrical potentials collected by the terminals.

5. The system according to claim 2, wherein each terminal of each acquisition system comprises at least one operational amplifier in order to measure the differences in potential between the analog electrical potentials that it collects and to transmit these differences in potential to the relay of this acquisition system, and wherein the relay of each acquisition system comprises a plurality of acquisition stages each connected at least to one of the terminals of this acquisition system in order to receive said analog electrical potential differences, each acquisition stage being connected to the connection unit and the power unit while being galvanically isolated from this power unit and from its connection unit.

6. The system according to one of claims 2 to 5, wherein the connection unit comprises a microcontroller arranged to serialize the digital signals converted by the analog-to-digital converters and transmitted by each of the acquisition stages to the connection unit into a single digital signal and to transmit this single digital signal to the central system.

7. The system according to one of the preceding claims, wherein the central system is connected to each acquisition system by a data transmission network, wherein the central system is arranged to transmit signals containing electrical power to each acquisition system via the data transmission network, and, wherein the connection unit is arranged to transmit the digital signals to the central system via said data transmission network, and wherein the power unit is arranged to extract the electrical power from the signals received from the central system via the data transmission network and to transmit said extracted electrical power to the operational amplifiers and to the analog-to-digital converters.

8. The system according to one of the preceding claims, wherein the connection unit of each acquisition system comprises a coupler arranged to transmit the digital signals converted by the analog-to-digital converters of this acquisition system to the connection unit, the coupler being arranged to galvanically isolate these analog-to-digital converters from the connection unit.

9. The system according to one of the preceding claims, wherein each acquisition system includes two rechargeable electric batteries and a switching system connected to the electric batteries, to the power unit, to the operational amplifiers and to the analog-to-digital converters of this acquisition system, the power unit of this acquisition system including a control unit for said switching system arranged to simultaneously:
• connect one of the electric batteries to the operational amplifiers and to the analog-to-digital converters of this acquisition system and disconnect this electric battery from the power unit; and
• disconnect the other of the electric batteries from the operational amplifiers and the analog-to-digital converters and connect this electric battery to the power unit.

10. The system according to one of the preceding claims, wherein the electrical energy source of the central system comprises a photovoltaic panel.
